# EUROPEAN PATENT SPECIFICATION

(11) **EP 0 565 548 B1**
(45) Date of publication and mention of the grant of the patent: **22.05.1996**
(21) Application number: 92901348.0
(22) Date of filing: 20.12.1991
(51) Int. Cl.: A61K 39/00, C12N 15/00, A61K 39/12

(54) **TWO-PHASE SYSTEM FOR THE PRODUCTION AND PRESENTATION OF FOREIGN ANTIGENS IN HYBRID LIVE VACCINES**
ZWEIPHASENSYSTEM ZUR HERSTELLUNG UND PRÄSENTATION VON FREMDANTIGENEN IN HYBRIDEN LEBENDIMPFSTOFFEN
SYSTEME A DEUX PHASES DE PRODUCTION ET DE PRESENTATION D'ANTIGENES ETRANGERS DANS LES VACCINS HYBRIDES VIVANTS

(30) Priority: 20.12.1990 DE 4041045
(43) Date of publication of application: 20.10.1993
(73) Proprietor: Max-Planck-Gesellschaft zur Förderung der Wissenschaften e.V., D-37073 Göttingen (DE)
(72) Inventor: MEYER, Thomas, D-7400 Tübingen (DE); POHLNER, Johannes, D-7400 Tübingen (DE); REUSS, Frank, U., D-8755 Alzenau (DE); YAN, Zhengxin, D-7400 Tübingen (DE)
(74) Representative: VOSSIUS & PARTNER
(86) International application number: PCT/EP91/02478
(87) International publication number: WO 92/11027

(56) References cited:
- EP-A- 0 024 956
- EP-A- 0 263 591

## Description

The method common in medicine of producing effective protection against infectious diseases in human beings and animals is based on the principle of active immunization by using pathogen-specific antigens. There are, for example, prophylactic vaccinations against a number of diseases in human beings which are caused by bacteria or viruses. However, vaccinations against fungi and parasites are possible in principle too.

The basis for the protective effect of such vaccines is that important antigens, which in general originate or are derived from the pathogen, are brought into contact with the immune system of human beings or animals by infection or another suitable administration so that a specific immune response directed to the administered antigens is induced. The aim and object of this is to select the administered antigens in such a way and to present them to the immune system such that the induced immune response is directed against the pathogen and a subsequent infection is thus prevented. The induced immune response can be of either humoral (based on antibodies) or cellular nature or both.

The vaccine containing or forming antigens can be constructed or composed in a different way (see Bloom, Nature, 342:115-120, 1989). A simple method consists in using killed pathogens as the vaccine. Improvements are often achieved by only employing a few isolated components of the pathogen in the vaccine which represent important antigens. Moreover, new vaccines often only contain a few well-defined (e.g. purified from the pathogen, or prepared by genetic engineering or otherwise) components which act as an antigen by suitable presentation. Furthermore, every combination of the cited possibilities is conceivable. The common factor of the vaccines is that they consist of inactivated antigen material.

In contrast to these inactivated vaccines, the further possibility of immunising with biologically intact pathogens (so-called live vaccines) and conveying effective protection has long been known. Vaccination with living viruses (Zanetti, Immunology Today, 8:18-25, 1987) and BCG bacteria (Lotte, Adv. Tuberc. Res. 32:107-193, 1984) fall under this category, for example, as well as oral immunisation with living Ty21a Salmonella (Germanier, J. Infect. Dis. 131:553-558, 1975). The principle of such live vaccines is based on the use of an attenuated (or immunologically related non-virulent for a certain species) pathogen strain which is able to cause infection and effective immunological protection against the actual pathogen but is no longer pathogenic per se. Experience in the application of live vaccines lies above all with viruses and bacteria; in principle, however, similar vaccines can also be developed on the basis of fungi and parasites. Live vaccines often have advantages over comparable inactivated vaccines because they e.g. convey better immunological protection and are safer and less expensive.

New developments have furthermore shown that it is possible to change live vaccines by genetic engineering so that they not only present their own antigens to the immune system but also additional antigens which are derived from a different pathogen species. With such hybrid live vaccines it is possible to achieve immunological protection not only against the pathogen from which the live vaccine is derived or with which it is related but also against pathogens against which the immune response to the additional antigen is directed. Depending on the species on which the live vaccine is based, one or more additional antigens can be presented to the immune system and immunological protection can thus be conveyed. This is already realizable in practice with viral and bacterial live vaccines (see Dougan, J. Gen. Microbiol. 135:1397-1406, 1989). In contrast to bacterial or other cellular live vaccines, it is however natural for viral live vaccines (as well as for viruses in general) to be able to express their genetic information only after infection of a cell. In this case the formation of additional antigen by a viral live vaccine consequently requires the infection of cells of the individual to be protected. The presentation of additional antigens to the immune system can on the one hand take place via the infected cell per se or on the other hand via the viral live vaccine which carries additional antigen in its packaging.

A considerable problem with the construction and use of such hybrid live vaccines is however the circumstance that the production of additional antigens often changes the biological properties of the live vaccine and destabilizes the immunological effect so that the desired immunological protection is not achieved or only to a reduced extent. This can be the case in particular if the additional antigen is produced in large quantities as would often be required for the induction of a good immune response, and/or if the additional antigen is otherwise toxic for the live vaccine itself. In other words: The hybrid live vaccine behaves differently to the original live vaccine with respect to the course of an infection and thus with respect to its immunization potential because it produces one or more additional antigens. This also means that, depending on the kind of additional antigens produced by the live vaccine, the infectious properties and the effectiveness of the immunisation of the live vaccine cannot be inferred in so far as it is at all possible to achieve an effective immune response.

Current experiments to solve this problem in bacterial systems pursue the goal of controlling the formation of additional antigens in a live vaccine by external influences, i.e. to bring the genes which code for the formation of additional antigens under the control of an inducible promoter. The additional antigens of the live vaccine would consequently only be formed in dependence on their external influences or the environment. Such external influences can be e.g. a certain substance or a certain temperature (e.g. lac system: De Boer, Proc. Natl. Acad. Sci. USA, 80:21-25, 1983; P₁-system: Remaut, Gene, 15:81-93, 1981). It would be ideal if the external influences required for the formation of the antigens were only to be present where the live vaccine came into contact with the immune system but not in the course of the infection process so that the infection process necessary for the immune response is not disturbed. However, this is hardly realisable in practice not only because the infection process and the initiation of contact with the immune system are biologically linked but also because the conditions at the site of action of the live vaccine, i.e. in certain areas of the body of the person to be vaccinated, can be purposefully controlled with the available means neither with respect to location nor with respect to time.

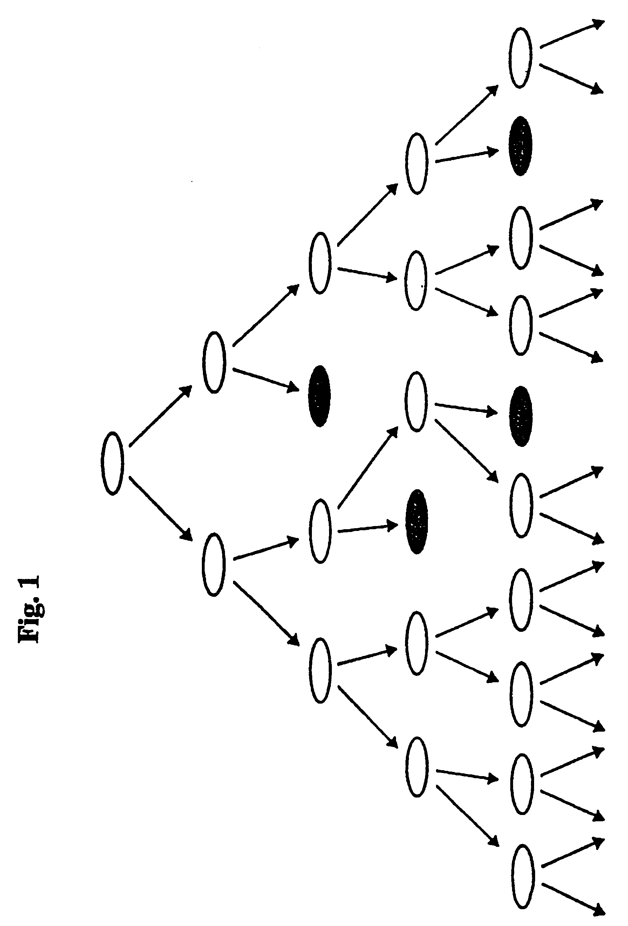

It is therefore the object of the present invention to introduce a genetic element into the construction of hybrid live vaccines which, on the one hand, allows the production of sufficiently large quantities of additional antigen at the immunological site of action and, on the other hand, does not interfere with the course of infection by the hybrid live vaccine and thus the expected immunological protection. This object was achieved by making the formation of the additional antigens of the hybrid live vaccine dependent on random genetic events which occur relatively frequently. This principle implies the existence of two subpopulations/phases which originate from the live vaccine, namely of the hybrid live vaccine itself (subpopulation/phase A) which does not produce any additional antigen and therefore reproduces without its properties changing in relation to the original strain and is capable of a normal course of infection and a normal immune response, and a subpopulation/phase B which may have lost these properties but is constantly newly regenerated from subpopulation A and releases large quantities of additional antigen at the site of action (see Fig. 1). Subpopulation A therefore has the task of guaranteeing a perfect course of infection while subpopulation B serves to build up an effective immune response to the additionally formed antigens.

This object is achieved by the embodiments of the appended claims.

Random events which lead to the formation of subpopulations are natural and can mostly be traced back to changes in the DNA, so-called (programmed) DNA reorganizations (Borst, Science, 235:658-667, 1987). In principle, all naturally-observed mechanisms of DNA reorganization can be used for the task set, provided that they can be reproduced suitably frequently in the hybrid live vaccine. The frequency of the formation of subpopulation B is preferably 0,1% to 50% per cell and cell generation; in particular cases, the frequency can, however, be higher or lower. Particularly suitable for application in a hybrid live vaccine are simple mechanisms of DNA reorganization which occur at specific sites, such as inversion (Craig, Cell, 41:649-650, 1985) or deletion by resolution of transposon cointegration (Grindley, Annual Rev. Biochem., 54:863-896, 1985) of a DNA segment. However, other site-specific DNA reorganisations or such DNA reorganisations which are based on slipped-strand-mispairing (Levinson, Mol. Biol. Evol. 4:203-221, 1987; Stern, Cell, 47:61-71, 1986) seem suitable for the cited object.

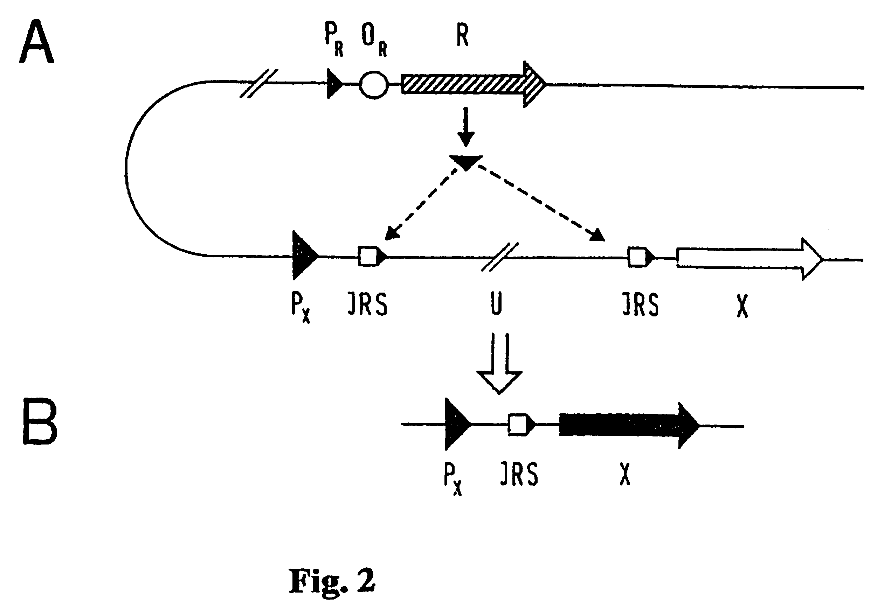

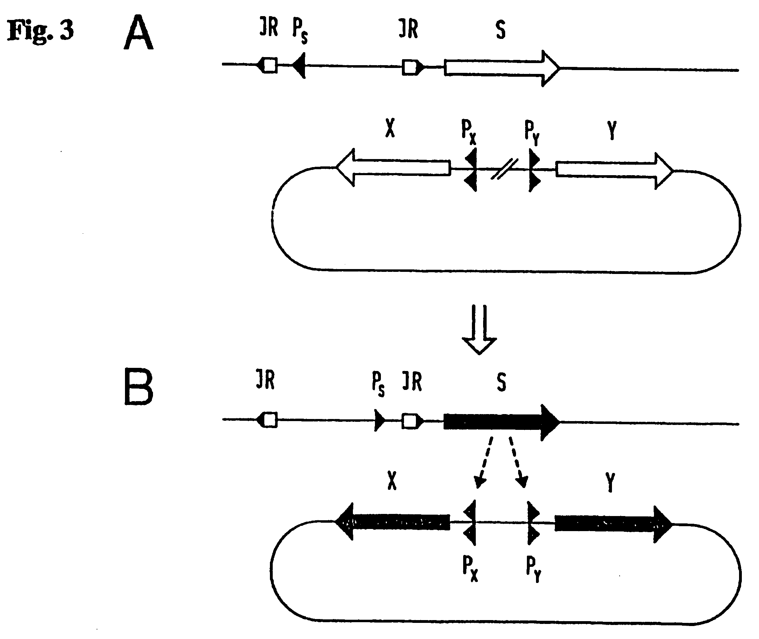

It must be the purpose of the cited spontaneously occurring DNA reorganisation in a live vaccine to lead directly or indirectly to the production of additional antigen, i.e. to the formation of subpopulation B which produces the antigen. This occurs very simply e.g. by positioning an expression signal (for example the promoter) of a gene by DNA reorganisation in front of the gene such that said gene changes from a non-expressed to an expressed state. All variants of this principle are possible; but they all have the goal of bringing about a change in the expression of a gene by DNA reorganisation (see Fig. 2 and Fig. 3). It usually makes sense to switch on genes by DNA reorganisation although the opposite is also possible.

The gene switched on by DNA reorganisation can, on the one hand (model 1), be the gene coding for an additional antigen or (if the additional gene is not a protein but an enzymatic synthesis product, e.g. a carbohydrate) a gene required for the antigen synthesis, or, on the other hand (model II), a gene encoding a protein which controls the expression of the actual gene coding for the antigen. Model I is thus a system which by DNA reorganisation directly codes for the synthesis of the additional antigen or for an enzyme required for the synthesis while model II represents a system which allows the production of the additional antigen via a cascade system (see Fig. 2 and Fig. 3). The cascade system can be realized e.g. in that the gene directly controlled by DNA reorganisation codes for an RNA polymerase which is specific for the promoter preceeding the gene coding for the antigen, or a gene regulator which in another specific manner induces the expression of the gene coding for the antigen (e.g. T7 polymerase: Studier, Meth. Enzymod. 185:60-89, 1990; lac system: De Boer, Proc. Natl. Acad. Sci. USA, 80:21-25, 1983). In this case too, there are all the possibilities of variation which nature offers. Whilst model I is on the one hand less complicated, the application of model II has advantages because high levels of expression can be achieved after one single DNA reorganization due to the increasing effect of the cascade. Furthermore, with this model several genes which code for different additional antigens within one hybrid live vaccine can be switched on at the same time.

The realization of the described system is technically particularly simple in bacterial live vaccines. The genetic element capable of DNA reorganisation can be held in bacteria e.g. on a plasmid or introduced in the genome by means of a phage, a transposon or by homologous recombination. In the case of the cascade model II, the antigen-encoding genes, which have special sites for the binding of the gene products of the element capable of DNA reorganisation, can be introduced in a similar manner by using conventional techniques.

Depending on the principle of the underlying DNA reorganisation enzymes (referred to here as "control enzymes") are necessary which have to be provided by the cell so that DNA reorganisation can take place at all. In the case of an inversion according to the principle of the phage Mu an invertase is necessary, for example, besides cellular factors (Kahmann, Cell, 41:771-780, 1985); in the case of a deletion corresponding to the mechanism of the resolution of transposon cointegration the enzyme resolvase is necessary, for example, (Reed, Cell, 25:713-719, 1981); the formation of replicative circles corresponding to the replication of filamentous phages requires inter alia the gene 2 product of the phage (Meyer, Nature, 296:828-832, 1982). These enzymes as well as the DNA structure at which they attack (referred to here as "target sites"; e.g.: Mertens, EMBO J., 7:1219-1227, 1988), offer a suitable basis from which to regulate the frequency with which DNA reorganisation forms the additional antigen and thus to determine the ratio of populations A and B. Manipulation of the expression of the control enzymes or the target sites by genetic engineering makes it possible to adjust this ratio exactly to the desired ratio of the populations. However, it is also conceivable to subject the control enzymes in turn to a superordinate regulatory control in order to change the ratio of populations A and B by external influences (e.g. in dependence on the temperature) (see Fig. 2). Here, too, there are possibilities of variation as desired and given in the state of molecular biology.

The conventional methods of molecular biology serve for the construction of genetic elements which undergo DNA reorganisation. Moreover, it is advantageous to firstly clone such genetic elements in cells which do not synthesise control enzymes in order to keep them stable for manipulation and construction purposes. After preparation such elements can be inserted into the genome of the live vaccine by the conventional methods of molecular biology or can be kept extrachromosomally as a plasmid. The same applies to the genes which code for the control enzymes as well as with respect to genes indirectly or directly coding for antigens in the case of model II.

The final hybrid live vaccine is administered in a suitable manner (e.g. by oral dosage or by injection) to the individual to be protected. The administered dose of the hybrid live vaccine usually corresponds to that for the corresponding non-hybrid live vaccine.

### Examples

### Example 1

### Construction of an invertable DNA element for the production of CT-B antigen (Vibrio cholerae toxin B - subunit) in Salmonella.

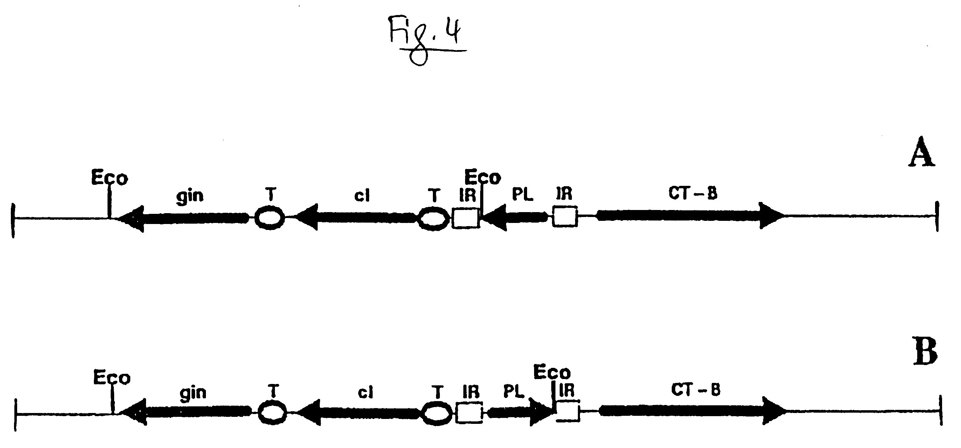

The genetic organisation of the invertable DNA element described here is represented in Figure 4. The element is contained on the plasmid pYZ17 and was constructed by using the following genes or other nucleotide sequences:

The gin gene is derived from the plasmid pLMugin-X16 (Mertens, EMBO J. 3:2415-2421, 1984) and was removed from there firstly as partially cleaved PvuI fragment which still contained the PvuI cleavage site contained in the gin gene itself as the only uncleaved PvuI cleavage site. This PvuI fragment was provided with EcoRI linkers at its ends and after cleavage with EcoRI and BamHI isolated as a BamHI/EcoRI fragment. This fragment was linked at the BamHI cleavage site with a synthetic BamHI/ClaI fragment (5'-GGATAAACCGATACAATTAAAGGCTCCTTTTGGAGCCTTTTTTTTTGGAGATTTTCAACG TG-3') of the terminator of bacteriophage fd so that a combinant EcoRI/ClaI fragment was obtained.

The cI857-gene fragment was isolated from the plasmid pRK248 (Bernard, Gene 5:59-76, 1979) by partial cleavage with HindII and subsequent ligation with an XhoI linker as an XhoI/Cla fragment.

The EcoRI/ClaI gin fragment was ligated to the cI857 gene fragment via the ClaI cleavage site and incorporated as an EcoRI/XhoI fragment into a derivative (pYZ13) of the plasmid pBT192 (Zahm, Mol. Gen. Genet., 194:188-194, 1984) which carries an additional (EcoRI/ClaI/XhoI/Bg1II) polylinker region.

The P₁ promoter was isolated as an XhoII fragment from the plasmid pLC2833 (Remaut, Gene 22:103-113, 1983) and linked via the BclI cleavage site at both ends with two synthetic BclI/XhoI oligonucleotides (5'-GATCATTTACCGTTTCCTGTAAACCGAGGTTTTGGATAAC-3') which correspond to the IR sequences (inverted repeats). The resulting fragment consisting of the sequence "IR-P₁-IR" was inserted into the singular XhoI cleavage of plasmid pfdA4 (Geider, Gene 33:341-349, 1985).

The rrnB T1 trancriptional terminator was obtained as a SalI/XhoI fragment with the nucleotide sequence XhoI

5'-
TCGAGGTAGCGAGCTTGAGGCATCAAATAAAACGAAAGGCTCAGTCGAAAGACTGGGC
CTTTCGTTTTATCTGTTGTTTGTCGGTGAACGCTCTCCTGAGTAGGACAAATCCGCCGGGAGCGGA
TTTGAACGTTGCGAAGCAACGGCCCGGAGGGTGGCGGGCAGGACGCCCGCCTTAAACTGCCACAAG
CTCGGTACCGTTAACG-3' SalI.

The promoterless CT-B encoding ctxB gene fragment was isolated from plasmid pTK1 (Klauser, EMBO J. 8:1991-1999, 1990) after cleavage with ClaI and subsequent linkage with a BglII linker as a BglII/SalI fragment.

The plasmid pYZ17 was constructed from the above-mentioned components in the following manner:
The SalI/XhoI T1 terminator fragment was inserted into the singular XhoI cleavage of plasmid pYZ13 and tested for the orientation of the remaining intact XhoI cleavage. site Derivatives from pYZ13 which carried the remaining XhoI cleavage site distally to the cI857 gene of pYZ13 were called pYZ15. The XhoI IR-P₁-IR fragment was inserted into the remaining XhoI cleavage site of pYZ16 so that the P₁ promoter was orientated to the cI857 gene. This plasmid (pYZ16) was identified by an asymmetric EcoRI cleavage (Fig. 4). Finally the neo^{r} gene contained in pYZ16 was replaced by the ctxB fragment by cleavage with BglII/SalI and subsequent ligation to give pYZ17 (Fig. 4). Since pYZ17 possesses a further EcoRI cleavage site downstream from gin besides the EcoRI cleavage site within the invertable P₁ promoter fragment, the orientation of the promoter can be determined very easily by cleavage with EcoRI, and the inversion of the promoters can be followed up very easily. The fractionation of the EcoRI cleavage products in an agarose gel results in two bands of defined size of phase A of plasmid pYZ17 corresponding to about 4960 bp and 1840 bp or two bands of defined size of phase B of the same plasmid corresponding to 4630 bp and 2170 bp.

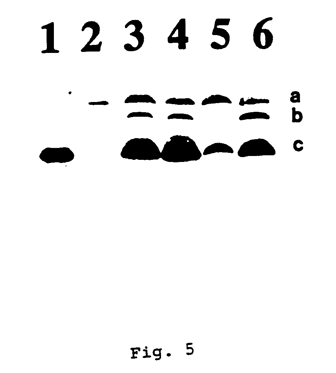

The construction and analysis of the described plasmids were carried out according to the methods described in Sambrock (Molecular Cloning, 2nd. ed., 1989, Cold Spring Harbor Laboratory Press). For explanation of the function of the invertable elements on plasmid pYZ17 see legend to Fig. 4. Fig. 5 shows evidence of the formation of CT-B antigen by the invertable element of plasmid pYZ17 in S.typhimurium SL3235 in comparison with plasmid pTK1 (Klauser, EMBO J., 9:1991-1999, 1990.

### Example 2

### Oral immunisation of BALB/c mice against CT-B antigen using a two-phase Salmonella typhimurium live vaccine

Salmonella typhimurium SL3235 (Hoiseth, Nature 291:238-239, 1981) was transformed according to the MgCl₂/CaCl₂ method (Lederberg, J. Bacteriol., 119:1072-1074, 1974) in separate preparations by plasmids pYZ17 and pTK1. The transformants were frozen in LB medium containing 15% glycerin and kept at -75°C. Before the immunisation experiment these strains were firstly incubated overnight on LB plates containing ampicillin (100 mg/ml) at 28°C. Single colonies were then inoculated in 10 ml liquid LB medium containing ampicillin (50 mg/ml) and cultivated overnight at 28°C. 5 ml of these overnight cultures were then added to 20 ml LB medium having a temperature of 28°C and containing ampicillin (50 mg/ml) and incubated for a further 4 hours at 28°C. The cultivated bacteria were subsequently harvested by centrifugation, washed in 10 ml saline (0.8% NaCl) and finally suspended in 2 ml saline.

The mice used for immunization (BALB/c) were given water but no food for 16 hours before the vaccination. All the mice were each given an oral dose of 0.2 ml 50% saturated Na₂HCO₃ solution. The mice were then divided up into groups of 5 and 30 minutes later were each given 0.2 ml of the prepared bacteria suspension administered gastrally with a blunt vaccination cannula: S.typhimurium SL3235 (group 1), S.typhimurium SL3235/pTK1 (group 2) or S.typhimurium SL3235/pYZ17 (group 3). The oral immunisation took place at 3 intervals (day 0, 7 and 14) with the same dosage each time. On day 20, serum and intestinal fluid were collected (Manning, FEMS Microbiol. Lett., 28:317-321, 1985). Protease inhibitor was added to the removed intestinal fluid (Elson, J. Immunol. Meth., 67:101-108, 1984). The samples of the mice were pooled according to the groups and determined by means of class-specific goat-anti-mouse antibodies in the ELISA test with respect to their specific antibody titer (Elson, J. Immunol. Meth., 67:101-108, 1984). The results are shown in Table 1.

### Figures and Tables

The present invention is illustrated by the following figures in combination with the description and the examples.

Fig. 1. Schematic representation of the spontaneous formation of two subpopulations/phases (A and B) of a cellular live vaccine.

The figure is an idealization of the exponential reproduction of one cell of the live vaccine (e.g. of a bacterium) which carries a genetic element according to the present invention which leads to the spontaneous formation of two subpopulations/phases A (open ellipses) and B (closed ellipses). While cells remain reproductive in phase A and therefore capable of infecting, spontaneously formed cells of phase B form large quantities of additional antigen which, on the one hand, leads to the induction of an additional immune response but, on the other hand, may inhibit the further reproduction of phase B. In the presented case the frequency of the formation of phase B is about 20%.

Fig. 2. Example of a specific DNA deletion in accordance with model I of the invention taking into account the role of the control enzyme.

X represents the antigen-encoding gene which is separated in phase A from its promoter (Pₓ) by a transcription terminating segment (U) and is therefore not expressed while in phase B the promoter is positioned directly in front of the gene X so that said gene is expressed. The deletion of the DNA segment U occurs by site-specific recombination at the sites IRS ("internal resolution sites" of the transposon Tn3) by the enzyme resolvase (TnpR of the transposon Tn3; here: closed, downwards pointing triangle). For this it is important that the two IRS sites whose nucleotide sequence is defined, are orientated in the same direction and are preferably localized within some 100 to 1000 base pairs on the same DNA molecule. The frequency of the deletion event can be determined by a number of factors, for example by slight sequence changes of one or both IRS sites or by the quantity of resolvase present in the cell. The quantity of this enzyme present in turn depends on the efficiency of the expression of the resolvase-encoding gene R. Provided that this gene in combination with its promoter (P_{R}) possesses an operator (O_{R}) on which transcription regulators can bind, it is possible in principle to regulate the frequency of the DNA reorganization (deletion of segment U) via the expression levels of the control-enzyme-encoding gene (R) by external influences, e.g. temperature.

Fig. 3 Example of a specific DNA inversion in accordance with model II of the invention.

The genes X and Y represent antigen-encoding genes which are contained e.g. on a plasmid of a hybrid bacterial live vaccine in any arrangement. Promoters (Pₓ and P_{y}) proceed these genes and are specific for certain RNA polymerases (e.g. the polymerase of phage T7) which are not formed by the live vaccines/bacterial cells themselves. In phase A the genes X and Y cannot be expressed since the specific polymerase is lacking. However, the live vaccine/bacterial cell contains the gene (S) which codes for this polymerase. The gene S can now be activated e.g. by DNA inversion whereby the promoter Pₛ is positioned directly in front of the polymerase gene S by the inversion of a segment so that said gene is expressed. The inversion of the DNA segment containing the promoter is catalyzed (e.g. in analogy to the G segment of the bacteriophage Mu) by an enzyme (gin/invertase) which attacks at well-defined target sites (IR/inverted repeats) which hold the opposite orientation on the DNA. The frequency with which the inversion of the fragment carrying the promoter occurs depends on several factors (inter alia the quantity of the invertase present in the cell and the structure of the IR sites at which it attacks) and can be individually adjusted or changed by manipulation by genetic engineering. In this example, the site-specific inversion of a DNA segment thus initiates a cascade which finally effects the activation of the antigen-encoding genes X and Y.

Fig. 4 Example of a genetic element according to the present invention for a bacterial live vaccine and the nature of its effect.

In the figure, the sketches A and B are the two phases of an invertable element, corresponding to how this can be present in the subpopulations A or B after introduction into a bacterial strain. The element shown essentially corresponds to model I (direct expression of the antigen gene) and possesses a superordinate regulatory control function which is dependent on the temperature as an external influence. In phase A the promoter P₁ (PL) responsible for the expression of the antigen gene is directed in the direction of the cI857 gene, which codes for a superordinate temperature sensitive repressor, and the gin gene, which codes for the control enzyme. The consequence of this is that a repressor which is able to function is formed at the permissive temperature of 28°C and reduces the transcription from the P₁ promoter (PL). At a higher temperature (e.g. 37°C) the transcription of the P₁ promoter (PL) is increased since the repressor is inactivated at least partially under such external influences. The temperature-dependent increase in the transcription also causes a corresponding increase in the expression of the following gin gene which as a control enzyme catalyses the inversion of the promoter at the target sites. Thus, the frequency of the inversion is also increased by a higher temperature and thus the transition of the invertable element in phase B. However, the inversion at a lowered temperature is not completely prevented since the gin gene is always expressed on a low level anyway and, moreover, because the host cell itself which is used as the live vaccine usually possesses an active gene similar to gin.

The inversion of the promoter causes less repressor and less control enzyme to be formed on the one hand and the antigen-encoding gene (CT-B) to be strongly expressed by means of the inverted promoter and due to the weaker formation of the cI repressor on the other hand.

In the above example, the invertable element is contained in the Salmonella typhimurium live vaccine (SL3235) on the plasmid pYZ17. Since this plasmid is present in several copies per bacterial cell, elements of both phase A and phase B can be present in a single bacterial cell. This results in additional interactions between the plasmids of phases A and B which cause a fractionation of the live vaccine not only in two subpopulations but also in the intermediates in between. If this is not desired, this can be easily taken care of by integration of the invertable element into the chromosome of the live vaccine or by cloning the element on a single copy plasmid.

For further functional analysis of the invertable element contained in plasmid pYZ17 on the plasmid pYZ17 see also Fig. 5 and Tab. 1. In the drawing the abbreviations mean as follows: Eco, EcoRI restriction sites for the analysis of the phase state of the invertable element; gin (also gin in the text), the gene coding for the control enzyme (invertase); T, transcription terminators for the reduction of the gene expression; cI (more precisely referred to in the text as cI857), the gene coding for the temperature-sensitive regulator (repressor); IR, the target sites of the invertase (inverted repeats); PL (also referred to in the text as P₁), the invertable promoter which is regulatable by the cI857 repressor; CT-B, the gene (ctxB) coding for the antigen (V.cholerae toxin B-subunit).

Fig. 5. This figure shows an immunoblot (Western) analysis of the formation of CT-B antigen in hybrid S.typhimurium SL3235 cells. In the figure, 20 ng of purified CT-B antigen obtained from Sigma is shown as a reference in lane 1 and 2.5 x 10⁷ cells corresponding to bacterial lysates in lanes 2 to 6; lane 2, S.typhimurium SL3235 cultured at 37°C; lane 3, S.typhimurium SL3235 (pTK1) cultured at 28°C; lane 4, S.typhimurium SL3235 (pTK1) cultured at 37°C; lane 5, S.typhimurium SL3235 (pYZ17) cultured at 28°C; lane 6, S.typhimurium SL3235 (pYZ17) cultured at 37°C. Position "a" refers to an unspecific cross reaction in the blot, position "b" to the precursor of the CT-B antigen and position "c" to the mature CT-B antigen. The samples are obtained and the immunoblot is performed according to Klauser (EMBO J., 9:1991-1999, 1990).

It is apparent that S.typhimurium SL3235 (pYZ17) produces very little CT-B antigen at 28°C in contrast to at 37°C. It is further evident that in contrast thereto the formation of CT-B antigen in the reference strain S.typhimurium SL3235 (pTK1) is independent of temperature and that in this strain in relation to S.typhimurium SL3235 (pYZ17), which was cultured at 37°C, less CT-B precursor protein is accumulated. The latter reflects the formation of little CT-B antigen in subpopulation A and much CT-B antigen in subpopulation B in S.typhimurium SL3235 (pYZ17) cultured at 37°C: The formation of much CT-B in only a few cells leads to a hold-up in the conversion from precursor into mature CT-B antigen.

Tab. 1 Humoral immune response after oral immunization of BALB/c mice with hybrid Salmonella typhimurium SL3235 strains.

The results of the experiment described in Example 2 are represented. The antibody titers stated therein correspond to those dilution levels of the samples which only just give a positive result in the ELISA test.

**Table I**

| | ELISA titer | | |
|---|---|---|---|
| | Serum | | Intestinal lavage |
| | IgG | IgA | IgG |
| SL3235 | 20 | 8 | 8 |
| SL3235 (pTK1) | 640 | 16 | 16 |
| SL3235 (pYZ17) | 10240 | 128 | 64 |

## Claims

1. A recombinant DNA for the production of a hybrid live vaccine comprising the following components:
- at least one gene encoding an antigen, a specific polymerase or a specific gene regulator;
- an expression signal; and
- an element being the result of a DNA reorganisation mechanism;
wherein the expression signal upon infection with the hybrid live vaccine which is capable of infection and immunogenically active per se (subpopulation A) is enabled by the spontaneous reorganisation of the DNA by the DNA reorganisation mechanism to control the expression of said gene so that subpopulation B of the hybrid live vaccine is regenerated from subpopulation A which produces additional antigen derived from a different pathogen species and acts immunogenicallv with respect to said additional antigen.

2. The recombinant DNA of claim 1, wherein said DNA reorganisation mechanism is a specific DNA inversion process.

3. The recombinant DNA of claim 1, wherein said DNA reorganisation mechanism is a specific DNA deletion process.

4. The recombinant DNA of claim 1, wherein said DNA reorganisation mechanism is a specific DNA replication process.

5. The recombinant DNA of claim 1, wherein said DNA reorganisation mechanism is a specific slipped-strand-mispairing mechanism.

6. The recombinant DNA of claim 1, wherein the DNA reorganisation is catalysed by a control enzyme.

7. The recombinant DNA of claim 6, wherein the frequency of the spontaneous DNA reorganisation is controlled superordinately by the regulation of the control enzyme.

8. The recombinant DNA of any one of claims 1 to 7, wherein the DNA reorganisation leads directly to the formation of additional antigen in subpopulation B.

9. The recombinant DNA of any one of claims 1 to 7, wherein the DNA reorganisation initiates a cascade reaction and leads indirectly to the formation of additional antigen in subpopulation B.

10. The recombinant DNA of claim 9, wherein the cascade is initiated by the formation of a specific polymerase, whereby the latter directly or indirectly causes the expression of the gene(s) encoding the antigen.

11. The recombinant DNA of claim 9, wherein the cascade is initiated by the formation of a specific gene regulator which directly or indirectly causes the expression of the gene(s) encoding the antigen.

12. A hybrid live vaccine comprising a recombinant DNA of any one of claims 1 to 11 and being capable of forming two subpopulations A and B, characterized in that the division of the live vaccine into said subpopulations occurs by spontaneous DNA reorganisation in the recombinant DNA of the live vaccine, wherein said subpopulation A is capable of infection and immunogenically active per se while subpopulation B, which is regenerated from subpopulation A, produces additional antigen(s) and acts immunogenically with respect to said additional antigen(s).

13. The hybrid live vaccine of claim 12, characterized in that it comprises two subpopulations A and B.

14. The hybrid live vaccine of claim 12 or 13, which is a cellular live vaccine.

15. The hybrid live vaccine of claim 12 or 13 which is a viral live vaccine and expressed after infection of a cell.

16. The hybrid live vaccine of claim 14 which contains more than one copy of the recombinant DNA.

17. The hybrid live vaccine of claim 14 or 16 wherein the recombinant DNA is contained in a plasmid.

18. The hybrid live vaccine of claim 14 or 16 wherein the recombinant DNA is contained in the chromosome.

19. The recombinant live vaccine of any one of claims 13 to 18 wherein subpopulation B occurs with a frequency of 0.1% to 50% per cell and cell generation.

20. The hybrid live vaccine of any one of claims 12, 13, 14, 16, 17, 18 or 19, wherein the cell is a bacterium or a eukaryotic cell, such as a fungal cell.

21. The hybrid live vaccine of claim 20, wherein said bacterium or eukaryotic cell is an attenuated pathogen.

22. The hybrid live vaccine of any one of claims 12 to 21 for the immunization of a mammal.

23. A method for the production of a hybrid live vaccine of any one of claims 12 to 22, wherein the live vaccine is divided into two subpopulations A and B, characterized in that the division of the live vaccine into said subpopulations occurs by spontaneous DNA reorganisation in the recombinant DNA of the live vaccine, wherein said subpopulation A is capable of infection and immunogenically active per se while subpopulation B, which is regenerated from subpopulation A, produces additional antigen(s) and acts immunogenically with respect to said additional antigen(s).

24. A process for the preparation of a recombinant DNA according to any one of claims 1 to 11 which comprises combining the following components:
- at least one gene encoding an antigen, a specific polymerase or a specific gene regulator;
- an expression signal; and
- an element being the result of a DNA reorganisation mechanism;
wherein the expression signal upon infection with the hybrid live vaccine which is capable of infection and immunogenically active per se (subpopulation A) is enabled by the spontaneous reorganisation of the DNA by the DNA reorganisation mechanism to control the expression of said gene so that subpopulation B of the hybrid live vaccine is regenerated from subpopulation A which produces additional antigen derived from a different pathogen species and acts immunogenically with respect to said additional antigen.

## Patentansprüche

1. Rekombinante DNA zur Erzeugung eines hybriden Lebendimpfstoffs mit den folgenden Komponenten:
- mindestens einem Gen, das ein Antigen codiert, einer spezifischen Polymerase oder einem spezifischen Gen-regulator;
- einem Expressionssignal; und
- einem Element als Ergebnis eines DNA-Reorganisationsmechanismus;
wobei das Expressionssignal nach einer Infektion mit dem infektionsfähigen und an sich immunogenen hybriden Lebendimpfstoff (Subpopulation A) durch die spontane Reorganisation der DNA mit Hilfe des DNA-Reorganisationsmechanismus in die Lage versetzt wird, die Expression des Gens zu bewirken, so daß die Subpopulation B des hybriden Lebendimpfstoffs von der Subpopulation A regeneriert wird, die zusätzliches, von einer anderen pathogenen Art stammendes Antigen produziert und in bezug auf das zusätzliche Antigen immunogen wirkt.

2. Rekombinante DNA nach Anspruch 1, wobei der DNA-Reorganisationsmechanismus ein spezifischer DNA-Inversionsvorgang ist.

3. Rekombinante DNA nach Anspruch 1, wobei der DNA-Reorganisationsmechanismus ein spezifischer DNA-Deletionsvorgang ist.

4. Rekombinante DNA nach Anspruch 1, wobei der DNA-Reorganisationsmechanismus ein spezifischer DNA-Replikationsvorgang ist.

5. Rekombinante DNA nach Anspruch 1, wobei der DNA-Reorganisationsmechanismus ein spezifischer "slipped-strand-mispairing"-Mechanismus ist.

6. Rekombinante DNA nach Anspruch 1, wobei die DNA-Reorganisation durch ein Steuerenzym katalysiert wird.

7. Rekombinante DNA nach Anspruch 6, wobei die Häufigkeit der spontanen DNA-Reorganisation durch Regulation des Steuerenzyms übergeordnet kontrolliert wird.

8. Rekombinante DNA nach einem der Ansprüche 1 bis 7, bei der die DNA-Reorganisation unmittelbar zur Bildung des zusätzlichen Antigens der Subpopulation B führt.

9. Rekombinante DNA nach einem der Ansprüche 1 bis 7, bei der die DNA-Reorganisation eine Kaskadenreaktion initiiert und mittelbar zur Bildung des zusätzlichen Antigens der Subpopulation B führt.

10. Rekombinante DNA nach Anspruch 9, bei der die Kaskade durch Bildung einer spezifischen Polymerase initiiert wird, wobei letztere unmittelbar oder mittelbar die Expression des/der das Antigen codierenden Gens(e) bewirkt.

11. Rekombinante DNA nach Anspruch 9, bei der die Kaskade durch Bildung eines spezifischen Genregulators initiiert wird, der unmittelbar oder mittelbar die Expression des/der das Antigen codierenden Gene(s) bewirkt.

12. Hybrider Lebendimpfstoff, der eine rekombinante DNA nach einem der Ansprüche 1 bis 11 enthält und zwei Subpopulationen A und B bilden kann, dadurch gekennzeichnet, daß die Aufspaltung des Lebendimpfstoffs in zwei Subpopulationen durch spontane DNA-Reorganisation in der rekombinanten DNA des Lebendimpfstoffs erfolgt, wobei die Subpopulation A infektionsfähig ist und an sich immunogen wirkt, während die Subpopulation B, die von der Subpopulation A regeneriert wird, zusätzliche(s) Antigen(e) produziert und in bezug auf diese(s) zusätzliche(n) Antigen(e) immunogen wirkt.

13. Hybrider Lebendimpfstoff nach Anspruch 12, dadurch gekennzeichnet, daß er zwei Subpopulationen A und B aufweist.

14. Hybrider Lebendimpfstoff nach Anspruch 12 oder 13, der ein zellulärer Lebendimpfstoff ist.

15. Hybrider Lebendimpfstoff nach Anspruch 12 oder 13, der ein viraler Lebendimpfstoff ist und nach Infektion einer Zelle exprimiert wird.

16. Hybrider Lebendimpfstoff nach Anspruch 14, der mehrere Kopien der rekombinanten DNA enthält.

17. Hybrider Lebendimpfstoff nach Anspruch 14 oder 16, bei dem die rekombinante DNA in einem Plasmid enthalten ist.

18. Hybrider Lebendimpfstoff nach Anspruch 14 oder 16, bei dem die rekombinante DNA im Chromosom enthalten ist.

19. Rekombinanter Lebendimpfsto£f nach einem der Ansprüche 13 bis 18, bei dem die Subpopulation B mit einer Häufigkeit von 0,1 % bis 50 % pro Zelle und Zellgeneration vorliegt.

20. Hybrider Lebendimpfstoff nach einem der Ansprüche 12, 13, 14, 16, 17, 18 oder 19, bei dem die Zelle ein Bakterium oder eine eukaryontische Zelle, wie eine Pilzzelle, ist.

21. Hybrider Lebendimpfstoff nach Anspruch 20, bei dem das Bakterium oder die eukaryontische Zelle ein attenuierter Krankheitserreger ist.

22. Hybrider Lebendimpfstoff nach einem der Ansprüche 12 bis 21 zur Immunisierung eines Säugers.

23. Verfahren zur Herstellung eines hybriden Lebendimpfstoffs nach einem der Ansprüche 12 bis 22, bei dem man den Lebendimpfstoff in zwei Subpopulationen A und B aufspaltet, dadurch gekennzeichnet, daß die Aufspaltung des Lebendimpfstoffs in zwei Subpopulationen durch spontane DNA-Reorganisation in der rekombinanten DNA des Lebendimpfstoffs erfolgt, wobei Subpopulation A infektionsfähig ist und an sich immunogen wirkt, während Subpopulation B, die von Subpopulation A regeneriert wird, zusätzliche(s) Antigen(e) produziert und in bezug auf diese(s) zusätzliche(n) Antigen(e) immunogen wirkt.

24. Verfahren zur Herstellung einer rekombinanten DNA nach einem der Ansprüche 1 bis 11, das die Kombination folgender Komponenten umfaßt:
- mindestens eines Gens, das ein Antigen codiert, einer spezifischen Polymerase oder eines spezifischen Genregulators;
- eines Expressionssignals; und
- eines Elements als Ergebnis des DNA-Reorganisationsmechanismus;
wobei das Expressionssignal nach einer Infektion mit dem infektionsfähigen und an sich immunogenen hybriden Lebendimpfstoff (Subpopulation A) durch die spontane Reorganisation der DNA mit Hilfe des DNA-Reorganisationsmechanismus in die Lage versetzt wird, die Expression des Gens zu bewirken, so daß die Subpopulation B des hybriden Lebendimpfstoffs von der Subpopulation A regeneriert wird, die zusätzliches, von einer anderen pathogenen Art stammendes Antigen produziert und in bezug auf das zusätzliche Antigen immunogen wirkt.

## Revendications

1. ADN recombinant pour la production d'un vaccin vivant hybride comprenant les composants suivants:
- au moins un gène codant pour un antigène, une polymérase spécifique ou un régulateur de gène spécifique;
- un signal d'expression; et
- un élément qui est le résultat d'un mécanisme de réorganisation de l'ADN;
dans lequel le signal d'expression lors de l'infection par le vaccin vivant hybride qui est capable d'infection et est immunogéniquement actif par lui-même (sous-population A) est rendu capable par la réorganisation spontanée de l'ADN par le mécanisme de réorganisation de l'ADN de commander l'expression dudit gène de manière que la sous-population B du vaccin vivant hybride soit régénérée à partir de la sous-population A qui produit un antigène additionnel dérivé d'une espèce pathogène différente et agit de façon immunogène vis-à-vis dudit antigène additionnel.

2. ADN recombinant de la revendication 1, dans lequel ledit mécanisme de réorganisation de l'ADN est un processus d'inversion spécifique de l'ADN.

3. ADN recombinant de la revendication 1, dans lequel ledit mécanisme de réorganisation de l'ADN est un processus de délétion spécifique d'ADN.

4. ADN recombinant de la revendication 1, dans lequel ledit mécanisme de réorganisation de l'ADN est un processus de réplication spécifique de l'ADN.

5. ADN recombinant de la revendication 1, dans lequel ledit mécanisme de réorganisation de l'ADN est un processus de mésappariement spécifique à glissement de brin.

6. ADN recombinant de la revendication 1, dans lequel la réorganisation de l'ADN est catalysée par une enzyme de commande.

7. ADN recombinant de la revendication 6, dans lequel la fréquence de la réorganisation spontanée de l'ADN est commandée par un élément d'ordre supérieur au moyen de la régulation de l'enzyme de commande.

8. ADN recombinant de l'une quelconque des revendications 1 à 7, dans lequel la réorganisation de l'ADN aboutit directement à la formation d'un antigène additionnel dans la sous-population B.

9. ADN recombinant de l'une quelconque des revendications 1 à 7, dans lequel la réorganisation de l'ADN amorce une réaction en cascade et aboutit indirectement à la formation d'un antigène additionnel dans la sous-population B.

10. ADN recombinant de la revendication 9, dans lequel la cascade est amorcée par la formation d'une polymérase spécifique, cette dernière provoquant directement ou indirectement l'expression du ou des gène(s) codant pour l'antigène.

11. ADN recombinant de la revendication 9, dans lequel la cascade est amorcée par la formation d'un régulateur de gène spécifique qui provoque directement ou indirectement l'expression du ou des gène(s) codant pour l'antigène.

12. Vaccin vivant hybride comprenant un ADN recombinant de l'une quelconque des revendications 1 à 11 et qui est capable de former deux sous-populations A et B, caractérisé en ce que la division du vaccin vivant en lesdites sous-populations s'effectue par réorganisation spontanée de l'ADN dans l'ADN recombinant du vaccin vivant, où ladite sous-population A est capable d'infection et est immunogéniquement active par elle-même tandis que la sous-population B, qui est régénérée à partir de la sous-population A, produit un ou plusieurs antigène(s) supplémentaire(s) et agit immunogéniquement vis-à-vis du(des)dit(s) antigène(s) additionnel(s).

13. Vaccin vivant hybride de la revendication 12, caractérisé en ce qu'il comprend deux sous-populations A et B.

14. Vaccin vivant hybride de la revendication 12 ou 13, qui est un vaccin vivant cellulaire.

15. Vaccin vivant hybride de la revendication 12 ou 13 qui est un vaccin vivant viral et est exprimé après infection d'une cellule.

16. Vaccin vivant hybride de la revendication 14 qui contient plus d'un exemplaire de l'ADN recombinant.

17. Vaccin vivant hybride de la revendication 14 ou 16 dans lequel l'ADN recombinant est contenu dans un plasmide.

18. Vaccin vivant hybride de la revendication 14 ou 16 dans lequel l'ADN recombinant est contenu dans le chromosome.

19. Vaccin vivant recombinant de l'une quelconque des revendications 13 à 18 dans lequel la sous-population B apparaît avec une fréquence de 0,1% à 50% par cellule et génération cellulaire.

20. Vaccin vivant hybride de l'une quelconque des revendications 12, 13, 14, 16, 17, 18 ou 19, dans lequel la cellule est une bactérie ou une cellule eucaryotique, comme une cellule fongique.

21. Vaccin vivant hybride de la revendication 20, dans lequel ladite bactérie ou cellule eucaryotique est un agent pathogène atténué.

22. Vaccin vivant hybride de l'une quelconque des revendications 12 à 21 pour l'immunisation d'un mammifère.

23. Procédé de production d'un vaccin vivant hybride de l'une quelconque des revendications 12 à 22, dans lequel le vaccin vivant est divisé en deux sous-populations A et B, caractérisé en ce que la division du vaccin vivant en lesdites sous-populations s'effectue par réorganisation spontanée de l'ADN dans l'ADN recombinant du vaccin vivant, où ladite sous-population A est capable d'infection et est immunogéniquement active par elle-même tandis que la sous-population B, qui est régénérée à partir de la sous-population A, produit un ou plusieurs antigène(s) additionnel(s) et agit immunogéniquement vis-à-vis du ou desdits antigène (s) additionnel(s).

24. Procédé de préparation d'un ADN recombinant selon l'une quelconque des revendications 1 à 11 dans lequel on combine les composants suivants:
- au moins un gène codant pour un antigène, une polymérase spécifique ou un régulateur de gène spécifique;
- un signal d'expression; et
- un élément qui est le résultat d'un mécanisme de réorganisation de l'ADN;
dans lequel le signal d'expression lors de l'infection par le vaccin vivant hybride qui est capable d'infection et est immunogéniquement actif par lui-même (sous-population A) est rendu capable par la réorganisation spontanée de l'ADN par le mécanisme de réorganisation de l'ADN de commander l'expression dudit gène de manière que la sous-population B du vaccin vivant hybride soit régénérée à partir de la sous-population A qui produit un antigène additionnel dérivé d'une espèce pathogène différente et agit de façon immunogène vis-à-vis dudit antigène additionnel.
